Europäisches Patentamt

(19) European Patent Office

Office européen des brevets

(11) Publication number: **0 085 967**
**B1**

(12) **EUROPEAN PATENT SPECIFICATION**

(45) Date of publication of patent specification: **23.07.86**

(21) Application number: **83101077.2**

(22) Date of filing: **04.02.83**

(51) Int. Cl.⁴: **A 61 N 1/05,** A 61 N 1/36

(54) **Cardiac pacing lead with biodegradable fixation means.**

(30) Priority: **08.02.82 US 347007**

(43) Date of publication of application:
**17.08.83 Bulletin 83/33**

(45) Publication of the grant of the patent:.
**23.07.86 Bulletin 86/30**

(84) Designated Contracting States:
**DE FR GB NL**

(56) References cited:
**DE-A-3 048 805**
**US-A-3 981 309**
**US-A-4 236 529**
**US-A-4 280 514**
**US-A-4 281 669**

(73) Proprietor: **Cordis Corporation**
**10555 West Flagler Street**
**Miami Florida 33102-5700 (US)**

(72) Inventor: **MacGregor, David C.**
**10421 S.W. 89th Avenue**
**Miami, Florida 33176 (US)**
Inventor: **Saulson, Stanley H.**
**15200 S.W. 88th Avenue**
**Miami, Florida 33157 (US)**

(74) Representative: **Kuhnen, Wacker & Partner**
**Schneggstrasse 3-5 Postfach 1729**
**D-8050 Freising (DE)**

# Description

Electrical monitoring and stimulation of heart action is well known and has been employed to counter a variety of heart dysfunctions. Such monitoring and stimulation requires a reliable means of attaching and maintaining proximity of a conducting electrode to the heart wall. This need arises, for example, in securing a pervenous cardiac pacing lead to the inside wall of the right ventricle. There have been many attempts to achieve such a means. One way is by bonding the electrode to the endocardium with an adhesive. The problem with such adhesive bonding is that it may not provide reliable anchoring of the stimulation electrode and may produce an adverse tissue reaction. Another way is by use of a smooth-surfaced harpoon-like device. Here, a temporary anchor is achieved by piercing the heart wall with an absorbable "harpoon" stored within the electrode.

A third way of attaching an electrode to the inner heart wall is by the use of a tined device as disclosed in US—A—4 236 529. Here, the electrode is held in proximity to the wall of the heart by inert tines which extend from the lead adjacent to the electrode and form an acute angle with the electrode body. These tines maintain the electrode in electrical contact with the heart tissue. The problem with this type of tined device is that over time, the tines will stimulate fibrotic tissue growth which will make later removal of the lead more difficult and which may interfere with the pacing threshold as the tines are typically quite close to the electrode's contact point. Additionally, even after the formation of fibrosis around the electrode, the mechanical stresses on the tines, due to myocardial contractions, can cause shifts in the electrode's position and/or additional tissue reaction.

US—A—4,281,669 provides novel cardio-vascular devices or implants (including pacemaker electrodes) which have biocompatibility and hence reduce thrombogenic problems. The pacemaker electrode embodiment is perferably in the form of a rigid, porous metal coating on a dense coherent metal substrate with a network of interconnected pores substantially uniformly distributed throughout the coating. The rigid nature of the metal coating, the strength of the substrate-coating interface and the strength of the particle-particle bond in the coating provide excellent strength and wear resistance characteristics. The formation of a thin, smooth, firmly attached tissue coating on the porous surface allows the electrode to be incorporated into the cardio-vascular system. This tissue coating is formed by a combination of colonization by nucleated cells circulating in the blood stream onto the porous surface and subsequent differentiation into other cell types plus true soft tissue ingrowth into the porous surface from adjacent body tissue thereby achieving a more secure attachment than has previously been the case.

Although the porous pacing electrode offers the advantage of improved blood tissue compatibility over a smooth pacing electrode, both require a period of several weeks to months to become firmly attached, during which time another means of attaching the lead to the heart wall is needed.

## Summary of the Invention

The problems of the prior art are overcome by the present invention, which provides a non-penetrating means for temporary attachment of a tissue stimulating lead to the surface of the tissue to be stimulated, said means being constituted of biodegradable material absorbable in the blood and adjacent tissue of the patient.

In a preferred form, the electrical stimulating member of a cardiac pacing lead has an adherent porous metal coating. The porous metal coating comprises metal particles joined to adjacent particles to define a plurality of connected interstitial pores uniformly distributed throughout the coating. A plurality of biodegradable fins adjacent to the electrode tip is the means for temporary attachment of the pacer lead to the surface of the heart. After surgical introduction, temporary fixation of the device is supplied by the fins. The device achieves permanent fixation by ingrowth of viable tissue into the interstices of the porous electrode. Such growth is blood and tissue compatible and involves very little scarring or fibrous tissue reaction. The process of permanent fixation occurs over a period of weeks during which time the fins are gradually absorbed into the blood and tissue, resulting in little, if any, fibrotic growth in the region of the electrode.

## Brief Description of the Drawings

Fig. 1 is a diagrammtic view of a heart with parts broken away showing a ventricular pacing lead and an atrial pacing lead at implant;

Fig. 2 is a diagrammatic view of a heart with parts broken away showing a ventricular lead and an atrial lead in their chronic state;

Fig. 3 is a side view of a distal lead assembly embodying the invention;

Fig. 4 is a end view of a distal lead assembly embodying the invention;

Fig. 5 is a sectional view of a distal lead assembly embodying the invention;

Fig. 6 is a cross-sectional view taken across section line 6 of Fig. 5; and

Fig. 7 is a cross sectional view taken along section line 7 of Fig 6.

## Description of Preferred Embodiment

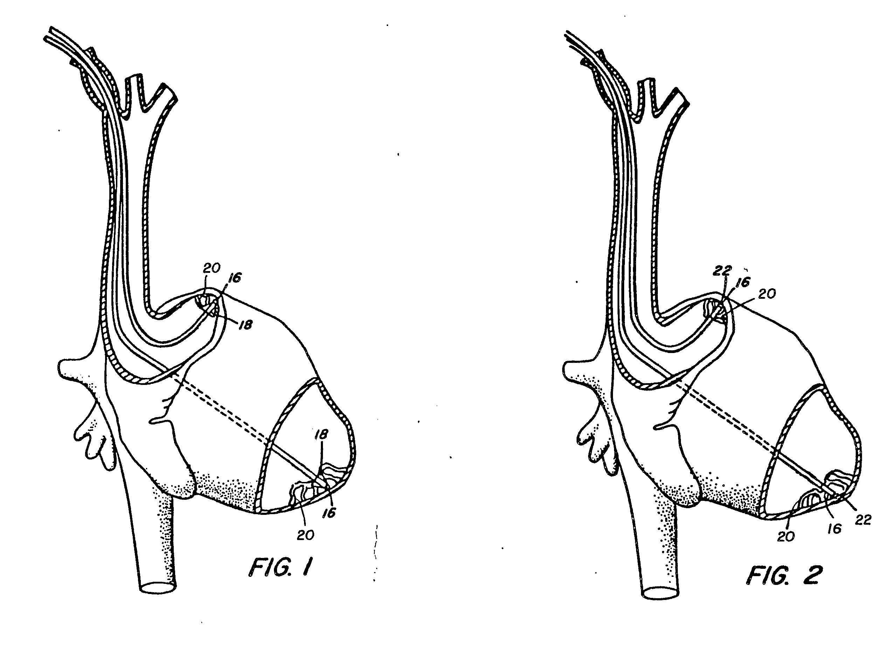

Fig. 1 is a diagrammatic view of the heart with parts broken away showing the atrial and ventricular leads at implant, with the porous electrode tips 16 in contact with the heart wall, the fins 18 ensnared in the trabeculi 20. Fig. 2 shows the chronic position of the leads after the fins have dissolved, the porous tips securing the leads, by a thin layer of fibrous tissue 22, which occurs within several weeks. As the porous electrode tip is "seen" by the heart as "friendly" and compatible, the resultant tissue growth minimizes the adverse reactions associated with the prior art. Thus, minimal tissue scarring and fibrous growth takes place to interfere with electrical transmission or to make subsequent removal difficult.

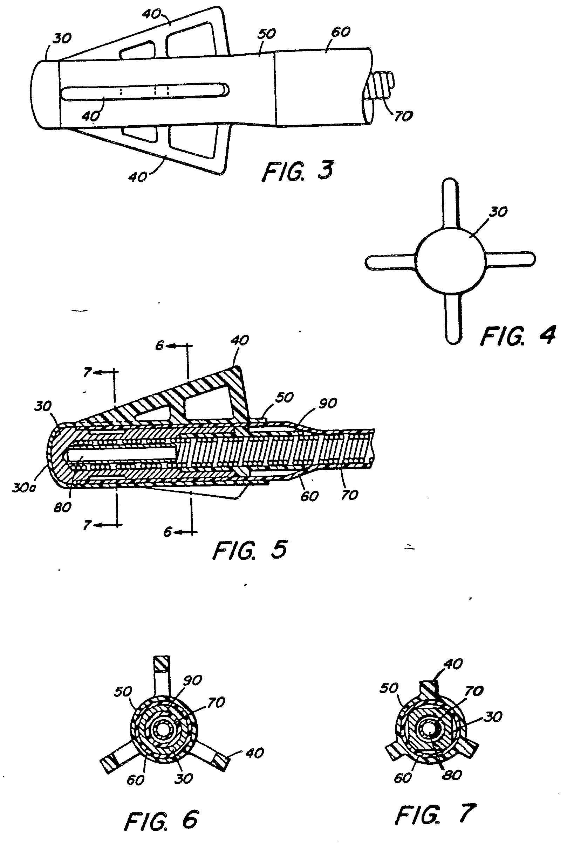

Referring now to Figs. 3—7, a preferred embodiment of the present invention, there is shown a fin member comprising fins 40 and cylindrical supporting portion 50. Absorbable non-conducting, pliable rearwardly-projecting fins 40 are situated in close proximity to the electrode tip, so as to enable them to temporarily hold the lead in place, yet not interfere with the growth of tissue at the tip. The actual temporary fixation means may be single or multiple and is not necessarily restricted to fins but could include other designs such as tines, balloons and helical coils.

Materials used for the fin member are similar or identical to those used for absorbable sutures in routine use in surgery, such as treated cat gut. The preferred material for the fin member is a copolymer of "Vicryl", a known suture material made by Ethicon consisting of a copolymer of glycolic and lactic acid, and polycaprolactone. Copolymerization with polycaprolactone serves to slow down degradation and to enhance flexibility.

The fins 40 are to be tapered and of ribbed design so that absorption occurs from their trailing edge tips inward toward the supporting portion 50 and forward toward the tip 30 so that loose pieces will not break off. The span of the fins will be small enough that, together with their pliable construction and tapered design, they will not interfere with implantation.

The metal electrode has a bulbous rounded "Elgiloy" (a metal alloy made by Elgiloy Company) tip 30 and an "Elgiloy" shank portion. The conducting tip 30 has a porous coating on its surface 30a, which coating consists of a layer of sintered "Elgiloy" beads. An alternate electrode material is platinum-iridium. Carbon can also be used as an electrode material, although metal is the preferred electrode material.

The resilient insulating sleeve 90 stretches over the coil and is positioned inside the electrode 30. The sleeve 90 serves to strengthen the joint and acts as a strain relief to protect the joint. The sleeve 90 is preferably made from a polyurethane, such as Pelethane™, but can be made from other materials such as silicone.

A flexible non-conducting polyurethane sheath 60 houses the "Elgiloy" coil 70 through which the stylet is inserted in the conventional manner. The sheath 60 extends over the length of the pacing lead. The sheath is expanded over the full length of the shank portion of the electrode and bonded in place. The sheath is then coated with a cyanoacrylate adhesive, superbonder 410 from Loctite Corp. The compression molded fin member is then slipped over the electrode into its place behind the tip of the electrode. An alternate approach is to stick the end of the electrode into a mold and mold the fins right onto it at that time. An alternate material for the sheath 60 is silicone rubber. Alternate materials for the coil 70 are other metals alloys and carbon.

The coil 70 has inside of it a metal staking pin 80 in order to crimp the shank around the coil without crushing the coil. The coil is inserted into the end of the electrode and the electrode is then crimped over the staking pin.

The coil 70 is in electrical conduct with the electrode tip 30. The electrical current flows from the pacer, typically implanted in the shoulder region (not shown) via the coil 70 to the tip 30.

The lead of this invention which has been described in reference to pacemaker applications is equally effective as a tissue stimulation lead for other stimulations within the body, such as stimulation of the central or peripheral nervous system.

While this invention has been described with reference to its preferred embodiment, other embodiments can achieve the same result. Variations and modifications of the present invention will be obvious to those skilled in the art.

## Claims

1. A Tissue stimulating lead, preferably for cardiac pacing, having at least one non-tissue-penetrating fixation means (18, 40) extending from the outer surface of said lead and a coating of porous material on a part (30a) of said surface, being characterized in that said non-tissue-penetrating fixation means (18, 40) are biodegradable for temporary attachment of said lead to the surface of the tissue to be stimulated.

2. The lead according to claim 1, being characterized in that said biodegradable fixation means (18, 40) is made from a copolymer of glycolic and lactic acid and polycaprolactone.

3. The lead according to claims 1 and 2, being characterized in that said biodegradable fixation means (18, 40) comprises a plurality of fins (18, 40).

## Patentansprüche

1. Gewebestimulierender Leiter, insbesondere für Herzschrittmacher, mit wenigstens einem Haltemittel (18, 40) welches das Gewebe nicht durchtritt und sich von der äußeren Oberfläche des Leiters erstreckt und mit einem Überzug aus porösem Material auf wenigstens einem Teil (30a) der Oberfläche, dadurch gekennzeichnet, daß die Haltemittel (18, 40), welche das Gewebe nicht durchtreten, für eine zeitweilige Befestigung des Leiters auf der Oberfläche des zu stimulierenden Gewebes biologisch abbaubar sind.

2. Leiter nach Anspruch 1, dadurch gekennzeichnet, daß die biologisch abbaubaren Haltemittel (18, 40) aus einem Copolymer aus Glycol- und Milchsäure sowie Polycaprolacton gefertigt sind.

3. Leiter nach Anspruch 1 oder 2, dadurch gekennzeichnet, daß die biologisch abbaubaren Haltemittel (18, 40) eine Mehrzahl von Flossen (18, 40) aufweisen.

## Revendications

1. Connexion de stimulation de tissu, de préférence pour la stimulation cardiaque, possédant au moins un dispositif de fixation (18, 40), ne pénétrant pas dans le tissu, qui s'etend depuis la surface extérieure de la connexion, et un revêtement de matériau poreux sur une partie (30a) de cette surface, caractérisée en ce que les dispositifs de fixation (18, 40), ne pénétrant pas dans le tissu, sont biodégradables et servant à l'attache temporaire de la connexion à la surface du tissu à stimuler.

2. Connexion selon la revendication 1, caractérisée en ce que le dispositif de fixation biodégradable (18, 40) est fait d'un copolymère d'acide glycolique et d'acide lactique et d'un polycaprolactone.

3. Connexion selon les revendications 1 et 2, caractérisée en ce que le dispositif de fixation biodégradable (18, 40) comporte plusieurs ailettes (18, 40).

FIG. 2

FIG. 1

FIG. 3

FIG. 4

FIG. 5

FIG. 6

FIG. 7